# EUROPEAN PATENT APPLICATION

(11) **EP 2 377 553 A1**
(43) Date of publication of application: **19.10.2011**
(21) Application number: 11152487.2
(22) Date of filing: 12.04.2007
(51) Int. Cl.: A61K 39/395, A61P 27/02

(54) **Use of IL-1 antibodies for treating ophthalmic disorders**

(30) Priority: 14.04.2006 US 792293 P; 12.05.2006 US 747134 P
(62) Divisional of application: 07755421.0
(71) Applicant: Novartis AG, 4056 Basel (CH); Novartis Pharma GmbH, 1230 Wien (AT)
(72) Inventor: Yoo, Dana Sue, Boston, MA 02134 (US); Neuner-Jehle, Martin, 4118, Rodersdorf (CH); Scassellati-Sforzolini, Baldo, Far Hills, NJ 07931 (US); Keating, Mark Taylor, Weston, MA 02493 (US); Markabi, Olivier Sabri, Summit, NJ 07901 (US)
(74) Representative: Vögeli-Lange, Regina

(57) **Abstract**

This invention relates to a novel uses of IL-1 compounds in the treatment and/or prevention of ophthalmic diseases or disorders and to methods of treating and/or preventing ophthalmic diseases or disorders in mammals, particularly humans.

## Description

This invention relates to a novel use of IL-1 receptor disrupting compounds (herein referred to as "IL-1 compounds"); such as small molecular compounds disrupting IL-1 receptor interaction, IL-1 antibodies or IL-1 receptor antibodies, e.g. IL-1 binding molecules described herein, e.g. antibodies disclosed herein, e.g. IL-1 binding compounds or IL-1 receptor binding compounds, and/or compounds decreasing protein levels of IL-1 receptor, in the treatment and/or prevention of ophthalmic diseases or disorders and to methods of treating and/or preventing ophthalmic diseases or disorders in mammals, including humans.

Interleukin-1 (IL-1), preferably Interleukin-1α (IL-1alpha or IL-1α or Interleukin-1α or Interleukin-1 alpha), more preferably Interleukin-β (IL-1beta or IL-1β or Interleukin-1beta or Interleukin-1β have the same meaning herein) is a potent immuno-modulator which mediates a wide range of immune and inflammatory responses. Typically, inappropriate or excessive production of IL-1 is associated with the pathology of various diseases and disorders, such as septicemia, septic or endotoxic shock, allergies, asthma, bone loss, ischemia, stroke, rheumatoid arthritis and other inflammatory disorders. For example, antibodies to IL-1β have been proposed for use in the treatment of IL-1 mediated diseases and disorders; see for instance, WO 95/01997 and the discussion in the introduction thereof and WO 02/16436, the content of which is incorporated by reference.

In accordance with the present invention, it has now surprisingly been found that IL-1 compounds are useful in the prevention and treatment of ophthalmic diseases or disorders in mammals, including humans.

As used herein, an IL-1 compound is an IL-1, IL-1α or IL-1β compound, preferably an IL-1α or IL-1β compound, and more preferably an IL-1β compound.

Ophthalmic diseases or disorders to be treated by IL-1 compounds, or treatable by IL-1 compounds are typically but not limited to wet age-related macular degeneration (wet AMD), dry age-related macular degeneration (dry AMD), diabetic macular edema (DME), cystoid macular edema (CME), non-proliferative diabetic retinopathy (NPDR), proliferative diabetic retinopathy (PDR), cystoid macular edema, vasculitis (e.g. central retinal vein occlusion), papilloedema, retinitis, conjunctivitis, uveitis, choroiditis, multifocal choroiditis, ocular histoplasmosis, blepharitis, dry eye (Sjögren's disease) and other ophthalmic diseases and disorders involving inflammation wherein the eye disease or disorder is associated with ocular neovascularization, vascular leakage, and/or retinal edema. Preferably the IL-1β compounds are useful in the prevention and treatment of wet AMD, dry AMD, CME, DME, NPDR, PDR, blepharitis, dry eye and uveitis, also preferably wet AMD, dry AMD, blepharitis, and dry eye, also preferably CME, DME, NPDR and PDR, also preferably blepharitis, and dry eye, in particular wet AMD and dry AMD, and also particularly wet AMD.

In the present description the terms "treatment" or "treatable" or "treat" refer to both prophylactic or preventative treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse.

In accordance with the particular findings of the present invention, the following embodiments are provided:

The present invention concerns compositions and methods for the prevention and treatment of ophthalmic diseases or disorders in mammals, including humans. Accordingly, the IL-1 compounds are also useful to prepare medicines and medicaments for the treatment ophthalmic diseases or disorders. In a specific aspect, such medicines and medicaments comprise a therapeutically effective amount of IL-1 compounds with a pharmaceutically acceptable carrier.

In another embodiment, the invention provides the use of an antibody which specifically binds to any of the above or below described polypeptides, e.g. IL-1 or IL-1 receptor, preferably IL-1, in the prevention and/or treatment of ophthalmic diseases or disorders such as those listed above, for example wet AMD, dry AMD, DME, NPDR, PDR, uveitis and other ophthalmic diseases and disorders involving inflammation.

As used herein an antibody is a monoclonal antibody, a humanized antibody, an antibody fragment or a single-chain antibody. Preferably the antibody concerns an isolated antibody which binds IL-1. Preferably, the antibody inhibits or neutralizes the activity of IL-1 (an antagonist antibody). Preferably, the antibody is a monoclonal antibody, which has either a human or nonhuman complementarily determining region (CDR) residue and a human framework region (FR) residue. The antibody may be labeled and may be immobilized on a solid support. Also preferably, the antibody is an antibody fragment, a monoclonal antibody, a single-chain antibody, or an anti-idiotypic antibody. More preferably, the present invention provides a composition for the contemplated uses comprising an IL-1 or IL-1 receptor antibody, preferably an IL-1 antibody, in a mixture with a pharmaceutically acceptable carrier. The composition comprises in particular a therapeutically effective amount of the antibody. Preferably, the composition is sterile. The composition may be administered in the form of a liquid pharmaceutical formulation, which may be preserved to achieve extended storage stability.

In a preferred embodiment, the invention provides the use of IL-1 compounds, e.g. IL-1 antibody, which are capable to interrupt the positive IL-1 feedback loop in vivo; in the prevention and/or treatment of ophthalmic diseases or disorders as contemplated above.

In a further embodiment, the invention concerns an article of manufacture, comprising:
(a) a composition of matter comprising an IL-1 or IL-1 receptor antibody, preferably an IL-1 antibody;
(b) a container containing said composition; and
(c) a label affixed to said container, or a package insert included in said container referring to the use of said IL-1 or IL-1 receptor antibody, preferably an IL-1 antibody, in the treatment of ophthalmic diseases or disorders, in particular those ophthalmic diseases or disorders contemplated above.

The composition may comprise a therapeutically effective amount of an IL-1 or IL-1 receptor antibody, preferably an IL-1antibody.

In yet a further embodiment, the invention provides a method or use as defined above, comprising co-administration of a therapeutically effective amount of IL-1 compounds in free form or salt form, preferably in a pharmaceutically acceptable delivery form such as intravenously or subcutaneously, and a second drug substance, said second drug substance being for example a VEGF (vascular endothelial growth factor) antagonist such as pegabtanib sodium, for example a VEGF-selective antibody such as bevacizumab, or a VEGF-selective antibody fragment such as ranibizumab.

In yet a further embodiment, an IL-1 compound used according to the invention is an IL-1 binding molecule which comprises an antigen binding site comprising at least one immunoglobulin heavy chain variable domain (V_{H}) which comprises in sequence hypervariable regions CDR1, CDR2 and CDR3, said CDR1 having the amino acid sequence Val-Tyr-Gly-Met-Asn, said CDR2 having the amino acid sequence Ile-Ile-Trp-Tyr-Asp-Gly-Asp-Asn-Gln-Tyr-Tyr-Ala-Asp-Ser-Val-Lys-Gly, and said CDR3 having the amino acid sequence Asp-Leu-Arg-Thr-Gly-Pro; and direct equivalents thereof.

In yet a further embodiment, an IL-1 compound used according to the invention is an IL-β binding molecule which comprise at least one immunoglobulin light chain variable domain (V_{L}) which comprises in sequence hypervariable regions CDR1', CDR2' and CDR3', said CDR1' having the amino acid sequence Arg-Ala-Ser-Gln-Ser-Ile-Gly-Ser-Ser-Leu-His said CDR2' having the amino acid sequence Ala-Ser-Gln-Ser-Phe-Ser and said CDR3' having the amino acid sequence His-Gln-Ser-Ser-Ser-Leu-Pro and direct equivalent thereof.

In yet a further embodiment, an IL-1 compound used according to the invention is a single domain IL-1β binding molecule comprising an isolated immunoglobulin heavy chain comprising a heavy chain variable domain (V_{H}) as defined above, e.g. for the preparation of a medicament for the treatment of ophthalmic diseases or disorders contemplated above.

In yet a further embodiment, an IL-1β compound used according to the invention is an IL-β binding molecule comprising both heavy (V_{H}) and light chain (V_{L}) variable domains in which said IL-1 binding molecule comprises at least one antigen binding site comprising:
a) an immunoglobulin heavy chain variable domain (V_{H}) which comprises in sequence hypervariable regions CDR1, CDR2 and CDR3, said CDR1 having the amino acid sequence Val-Tyr-Gly-Met-Asn, said CDR2 having the amino acid sequence Ile-Ile-Trp-Tyr-Asp-Gly-Asp-Asn-Gln-Tyr-Tyr-Ala-Asp-Ser-Val-Lys-Gly, and said CDR3 having the amino acid sequence Asp-Leu-Arg-Thr-Gly-Pro, and
b) an immunoglobulin light chain variable domain (V_{L}) which comprises in sequence hypervariable regions CDR1', CDR2' and CDR3', said CDR1' having the amino acid sequence Arg-Ala-Ser-Gln-Ser-Ile-Gly-Ser-Ser-Leu-His, said CDR2' having the amino acid sequence Ala-Ser-Gln-Ser-Phe-Ser, and said CDR3' having the amino acid sequence His-Gln-Ser-Ser-Ser-Leu-Pro ;
and direct equivalents thereof.

Unless otherwise indicated, any polypeptide chain is herein described as having an amino acid sequence starting at the N-terminal extremity and ending at the C-terminal extremity.
When the antigen binding site comprises both the V_{H} and V_{L} domains, these may be located on the same polypeptide molecule or, preferably, each domain may be on a different chain, the V_{H} domain being part of an immunoglobulin heavy chain or fragment thereof and the V_{L} being part of an immunoglobulin light chain or fragment thereof.

As used herein an "IL-1β binding molecule" refers to any molecule capable of binding to IL-1β either alone or associated with other molecules. The binding reaction may be shown or verified by standard methods (qualitative assays) including, for example, a bioassay for determining the inhibition of IL-1β binding to its receptor or any kind of binding assays, with reference to a negative control test in which an antibody of unrelated specificity but of the same isotype, e.g. an anti-CD25 antibody, is used. Advantageously, the binding of the IL-1β binding molecules may be shown in a competitive binding assay.

In analogy to the foregoing, an "IL-1α binding molecule" refers to any molecule capable of binding to IL-1α either alone or associated with other molecules, or an "IL-1 binding molecule" refers to any molecule capable of binding to IL-1 either alone or associated with other molecules.

Examples of antigen binding molecules include antibodies as produced by B-cells or hybridomas and chimeric, CDR-grafted or human antibodies or any fragment thereof, e.g. F(ab')₂ and Fab fragments, as well as single chain or single domain antibodies.

A single chain antibody consists of the variable domains of the heavy and light chains of an antibody covalently bound by a peptide linker usually consisting of from 10 to 30 amino acids, preferably from 15 to 25 amino acids. Therefore, such a structure does not include the constant part of the heavy and light chains and it is believed that the small peptide spacer should be less antigenic than a whole constant part. By "chimeric antibody" is meant an antibody in which the constant regions of heavy or light chains or both are of human origin while the variable domains of both heavy and light chains are of non-human (e.g. murine) origin or of human origin but derived from a different human antibody. By "CDR-grafted antibody" is meant an antibody in which the hypervariable regions (CDRs) are derived from a donor antibody, such as a non-human (e.g. murine) antibody or a different human antibody, while all or substantially all the other parts of the immunoglobulin e.g. the constant regions and the highly conserved parts of the variable domains, i.e. the framework regions, are derived from an acceptor antibody, e.g. an antibody of human origin. A CDR-grafted antibody may however contain a few amino acids of the donor sequence in the framework regions, for instance in the parts of the framework regions adjacent to the hypervariable regions. By "human antibody" is meant an antibody in which the constant and variable regions of both the heavy and light chains are all of human origin, or substantially identical to sequences of human origin, not necessarily from the same antibody and includes antibodies produced by mice in which the murine immunoglobulin variable and constant part genes have been replaced by their human counterparts, e.g. as described in general terms in EP 0546073 B1, USP 5545806, USP 5569825, USP 5625126, USP 5633425, USP 5661016, USP 5770429, EP 0 438474 B1 and EP 0 463151 B1.

Particularly preferred IL-1β binding molecules of the invention are human antibodies especially the ACZ 885 antibody as hereinafter described in the Examples and in WO 02/16436.

Thus in preferred antibodies of the invention, the variable domains of both heavy and light chains are of human origin, for instance those of the ACZ 885 antibody which are shown in SEQ ID NO: 1 and SEQ ID NO:2. The constant region domains preferably also comprise suitable human constant region domains, for instance as described in "Sequences of Proteins of Immunological Interest", Kabat E.A. et al, US Department of Health and Human Services, Public Health Service, National Institute of Health.

Hypervariable regions may be associated with any kind of framework regions, though preferably are of human origin. Suitable framework regions are described in Kabat E.A. et al, ibid. The preferred heavy chain framework is a human heavy chain framework, for instance that of the ACZ 885 antibody which is shown in SEQ ID NO: 1. It consists in sequence of FR1, FR2, FR3 and FR4 regions. In a similar manner, SEQ ID NO:2shows the preferred ACZ 885 light chain framework which consists, in sequence, of FR1', FR2', FR3' and FR4' regions.

Accordingly, the invention also provides an IL-1β binding molecule which comprises at least one antigen binding site comprising either a first domain having an amino acid sequence substantially identical to that shown in SEQ ID NO:1 starting with the amino acid at position 1 and ending with the amino acid at position 118 or a first domain as described above and a second domain having an amino acid sequence substantially identical to that shown in SEQ ID NO:2, starting with the amino acid at position 1 and ending with the amino acid at position 107.

Monoclonal antibodies raised against a protein naturally found in all humans are typically developed in a non-human system e.g. in mice, and as such are typically non-human proteins. As a direct consequence of this, a xenogenic antibody as produced by a hybridoma, when administered to humans, elicits an undesirable immune response which is predominantly mediated by the constant part of the xenogenic immunoglobulin. This clearly limits the use of such antibodies as they cannot be administered over a prolonged period of time. Therefore it is particularly preferred to use single chain, single domain, chimeric, CDR-grafted, or especially human antibodies which are not likely to elicit a substantial allogenic response when administered to humans.

In view of the foregoing, a more preferred IL-1β binding molecule of the invention is selected from a human IL-1β antibody which comprises at least;
a) an immunoglobulin heavy chain or fragment thereof which comprises (i) a variable domain comprising in sequence the hypervariable regions CDR1, CDR2 and CDR3 and (ii) the constant part or fragment thereof of a human heavy chain; said CDR1 having the amino acid sequence Val-Tyr-Gly-Met-Asn, said CDR2 having the amino acid sequence Ile-Ile-Trp-Tyr-Asp-Gly-Asp-Asn-Gln-Tyr-Tyr-Ala-Asp-Ser-Val-Lys-Gly, and said CDR3 having the amino acid sequence Asp-Leu-Arg-Thr-Gly-Pro and
b) an immunoglobulin light chain or fragment thereof which comprises (i) a variable domain comprising in sequence the hypervariable regions and optionally also the CDR1', CDR2', and CDR3' hypervariable regions and (ii) the constant part or fragment thereof of a human light chain, said CDR1' having the amino acid sequence Arg-Ala-Ser-Gln-Ser-Ile-Gly-Ser-Ser-Leu-His, said CDR2' having the amino acid sequence Ala-Ser-Gln-Ser-Phe-Ser, and said CDR3' having the amino acid sequence His-Gln-Ser-Ser-Ser-Leu-Pro;
and direct equivalents thereof.

Alternatively, an IL-1β binding molecule of the invention may be selected from a single chain binding molecule which comprises an antigen binding site comprising
a) a first domain comprising in sequence the hypervariable regions CDR1, CDR2 and CDR3, said CDR1 having the amino acid sequence Val-Tyr-Gly-Met-Asn, said CDR2 having the amino acid sequence Ile-Ile-Trp-Tyr-Asp-Gly-Asp-Asn-Gln-Tyr-Tyr-Ala-Asp-Ser-Val-Lys-Gly, and said CDR3 having the amino acid sequence Asp-Leu-Arg-Thr-Gly-Pro,
b) A second domain comprising the hypervariable regions CDR1', CDR2' and CDR3', said CDR1' having the amino acid sequence Arg-Ala-Ser-Gln-Ser-Ile-Gly-Ser-Ser-Leu-His, said CDR2' having the amino acid sequence Ala-Ser-Gln-Ser-Phe-Ser, and said CDR3' having the amino acid sequence His-Gln-Ser-Ser-Ser-Leu-Pro and
c) a peptide linker which is bound either to the N-terminal extremity of the first domain and to the C-terminal extremity of the second domain or to the C-terminal extremity of the first domain and to the N-terminal extremity of second domain;
and direct equivalents thereof.

As it is well known, minor changes in an amino acid sequence such as deletion, addition or substitution of one, a few or even several amino acids may lead to an allelic form of the original protein which has substantially identical properties.

Thus, by the term "direct equivalents thereof is meant either any single domain IL-1β binding molecule (molecule X),
(i) in which the hypervariable regions CDR1, CDR2 and CDR3 taken as a whole are at least 80% homologous, preferably at least 90% homologous, more preferably at least 95% homologous to the hypervariable regions as shown above and,
(ii) which is capable of inhibiting the binding of IL-1β to its receptor substantially to the same extent as a reference molecule having framework regions identical to those of molecule X but having hypervariable regions CDR1, CDR2 and CDR3 identical to those shown in above,
or any IL-1β binding molecule having at least two domains per binding site (molecule X'),
(i) in which the hypervariable regions CDR1, CDR2, CDR3, CDR1', CDR2' and CDR3' taken as a whole are at least 80% homologous, preferably at least 90% homologous, more preferably at least 95% homologous, to the hypervariable regions as shown above, and
(ii) which is capable of inhibiting the binding of IL-1β to its receptor substantially to the same extent as a reference molecule having framework regions and constant parts identical to molecule X', but having hypervariable regions CDR1, CDR2, CDR3, CDR1', CDR2' and CDR3', identical to those shown above.

In a further aspect the invention also provides an IL-1β binding molecule comprising both heavy (V_{H}) and light chain (V_{L}) variable domains in which said IL-1β binding molecule comprises at least one antigen binding site comprising:
a) an immunoglobulin heavy chain variable domain (V_{H}) which comprises in sequence hypervariable regions CDR1, CDR2 and CDR3, said CDR1 having the amino acid sequence Ser-Tyr-Trp-Ile-Gly, said CDR2 having the amino acid sequence Ile-Ile-Tyr-Pro-Ser-Asp-Ser-Asp-Thr-Arg-Tyr-Ser-Pro-Ser-Phe-Gln-Gly, and said CDR3 having the amino acid sequence Tyr-Thr-Asn-Trp-Asp-Ala-Phe-Asp-Ile, and
b) an immunoglobulin light chain variable domain (V_{L}) which comprises a CDR3' hypervariable region having the amino acid sequence Gln-Gln-Arg-Ser-Asn-Trp-Met-Phe-Pro;
and direct equivalents thereof.

In further aspect the invention provides an IL-1β binding molecule comprising both heavy (V_{H}) and light (V_{L}) chain variable domains in which said IL-1β binding molecule comprises at least one antigen binding site comprising:
a) an immunoglobulin heavy chain variable domain (V_{H}) which comprises in sequence hypervariable regions CDR1, CDR2 and CDR3, said CDR1 having the amino acid sequence Ser-Tyr-Trp-Ile-Gly, said CDR2 having the amino acid sequence Ile-Ile-Tyr-Pro-Ser-Asp-Ser-Asp-Thr-Arg-Tyr-Ser-Pro-Ser-Phe-Gln-Gly, and said CDR3 having the amino acid sequence Tyr-Thr-Asn-Trp-Asp-Ala-Phe-Asp-Ile, and
b) an immunoglobulin light chain variable domain (V_{L}) which comprises in sequence hypervariable regions CDR1', CDR2' and CDR3', said CDR1' having the amino acid sequence Arg-Ala-Ser-Gln-Ser-Val-Ser-Ser-Tyr-Leu Ala, said CDR2' having the amino acid sequence Asp-Ala-Ser-Asn-Arg-Ala-Thr, and said CDR3' having the amino acid sequence Gln-Gln-Arg-Ser-Asn-Trp-Met-Phe-Pro;
and direct equivalents thereof.

In the present description amino acid sequences are at least 80% homologous to one another if they have at least 80% identical amino acid residues in a like position when the sequence are aligned optimally, gaps or insertions in the amino acid sequences being counted as non-identical residues.

The inhibition of the binding of IL-1β to its receptor may be conveniently tested in various assays including such assays are described in WO 02/16436. By the term "to the same extent" is meant that the reference and the equivalent molecules exhibit, on a statistical basis, essentially identical IL-1β binding inhibition curves in one of the assays referred to above. For example, in IL-1β binding molecules of the invention typically have IC₅₀s for the inhibition of the binding of IL-1β to its receptor which are within +/-x5 of that of, preferably substantially the same as, the IC₅₀ of the corresponding reference molecule when assayed as described above.

For example, the assay used may be an assay of competitive inhibition of binding of IL-1β by soluble IL-1 receptors and the IL-1β binding molecules of the invention.

Most preferably, the IL-1β binding molecule for use according to the invention is an human IL-1 antibody which comprises at least;
a) one heavy chain which comprises a variable domain having an amino acid sequence substantially identical to that shown in SEQ ID NO:1 starting with the amino acid at position 1 and ending with the amino acid at position 118 and the constant part of a human heavy chain; and
b) one light chain which comprises a variable domain having an amino acid sequence substantially identical to that shown in SEQ ID NO:2 starting with the amino acid at position 1 and ending with the amino acid at position 107 and the constant part of a human light chain.

Most preferably, the IL-1β binding molecule for use according to the invention is ACZ885 (see Example).

The constant part of a human heavy chain may be of the γ₁, γ₂, γ₃, γ₄, µ, α₁, α₂, δ or ε type, preferably of the γ type, more preferably of the γ₁ type, whereas the constant part of a human light chain may be of the κ or λ type (which includes the λ₁, λ₂ and λ₃ subtypes) but is preferably of the κ type. The amino acid sequences of all these constant parts are given in Kabat et al ibid.

An IL-1β binding molecule of the invention may be produced by recombinant DNA techniques as e.g. described in WO 02/16436.

In yet another embodiment of the invention, IL-1β Compounds may be antibodies which have binding specificity for the antigenic epitope of human IL-1β which includes the loop comprising the Glu 64 residue of mature human IL-1β (Residue Glu 64 of mature human IL-1β correspond to residue 180 of the human IL-1β precursor). This epitope is outside the recognition site of the IL-1β receptor and it is therefore most surprising that antibodies to this eptitope, e.g. the ACZ 885 antibody, are capable of inhibiting the binding of IL-1β to its receptor. Thus the use of such antibodies for the treatment of ophthalmic diseases or disorders contemplated above is novel and are included within the scope of the present invention.

Thus in a further aspect the invention includes the use of an antibody to IL-1β which has antigen binding specificity for an antigenic epitope of human IL-1β which includes the loop comprising residue Glu 64 of mature human IL-1β and which is capable of inhibiting the binding of IL-1β to its receptor for the treatment of ophthalmic diseases or disorders contemplated above.

In yet further aspects the invention includes:
i) use of an antibody to IL-1β, which has antigen binding specificity for an antigenic epitope of mature human IL-1β which includes the loop comprising Glu 64 and which is capable of inhibiting the binding of IL-1β to its receptor, for the prevention and/or treatment of ophthalmic diseases or disorders contemplated above,
ii) a method for the prevention and/or treatment of ophthalmic diseases or disorders contemplated above in a patient which comprises administering to the patient an effective amount of an antibody to IL-1β which has antigen binding specificity for an antigenic epitope of mature human IL-1β which includes the loop comprising Glu 64 and which is capable of inhibiting the binding of IL-1β to its receptor;
iii) a pharmaceutical composition comprising an antibody to IL-1β which has antigen binding specificity for an antigenic epitope of mature human 1L-1β which includes the loop comprising Glu 64 and which is capable of inhibiting the binding of IL-1β to its receptor, in combination with a pharmaceutically acceptable excipient, diluent or carrier; for the treatment of ophthalmic diseases or disorders contemplated above,
iv) use of an antibody to IL-1β which has antigen binding specificity for an antigenic epitope of mature human IL-1β which includes the loop comprising Glu 64 and which is capable of inhibiting the binding of IL-1β to its receptor, for the preparation of a medicament for the treatment of ophthalmic diseases or disorders contemplated above.

For the purposes of the present description an antibody is "capable of inhibiting the binding of IL-1β" if the antibody is capable of inhibiting the binding of IL-1β to its receptor substantially to the same extent as the ACZ 885 antibody, i.e. has a dissociation equilibrium constant (K_{D}) measured e.g. in a standard BIAcore analysis as disclosed in the Example of 10nM or lower, e.g. 1 nM or lower, preferably 100 pM or lower, more preferably 50 pM or lower.

Thus in a yet further aspect the invention provides the use of an antibody to IL-1β which has a KD for binding to IL-1β of about 10 nM, 1 nM, preferably 100 pM, more preferably 50 pM or less for the treatment of ophthalmic diseases or disorders contemplated above. This aspect of the invention also includes uses methods and compositions for such high affinity antibodies, as described above for antibodies to IL-1β have binding specificity for an antigenic determinant of mature human IL-1β which includes the loop comprising Glu 64.

IL-1β binding molecules as defined above, in particular IL-1β binding molecules according to the first and second aspects of the invention antibodies which have binding specificity for the antigenic epitope of mature human IL-1β which includes the loop comprising Glu 64, in particular antibodies which are capable of inhibiting the binding of IL-1β to its receptor; and antibodies to IL-1β which have a K_{D} for binding to IL-1β of about about 10 nM, 1 nM, preferably 100 pM, more preferably 50 pM or less are herein referred to as antibodies of the invention.

In yet another embodiment of the invention, the further uses of the IL-1β compounds, e.g. the antibodies of the invention are as follows:
Prevention and treatment of ophthalmic diseases or disorders contemplated above.

For all indications disclosed herein, the appropriate dosage will, of course, vary depending upon, for example, the particular IL-1β compound, e.g. the antibody of the invention to be employed, the host, the mode of administration and the nature and severity of the condition being treated. However, in prophylactic use, satisfactory results are generally indicated to be obtained at dosages from about 0.05 mg to about 10 mg per kilogram body weight more usually from about 0.1 mg to about 5 mg per kilogram body weight. An antibody of the invention is conveniently administered parenterally, intravenously, e.g. into the antecubital or other peripheral vein, intramuscularly, or subcutaneously. In addition, antibody of the invention may be administered locally, e.g. direct intravitreal injection, subconjunctival injection, subtenon injection, peribulbar injections, intra-ocular implantable devices and topical (eye drop or ointment) application.

In yet another embodiment, the invention concerns a surprising frequency of dosing for therapeutic uses, i.e. the treatment schedule with IL-1β compounds, preferably IL-1β antibodies, more preferably ACZ885 (at a typical dose, e.g. between about 0.1 mg to about 50 mg, more preferably between 0.5 mg to 20 mg, even more preferably from 1 mg to 10 mg, of ACZ885 per kg body weight of the patient) may be once every week or less frequently, more preferably once every 2 weeks or less frequently, more preferably once every 3 weeks or less frequently, more preferably once every month or less frequently, more preferably once every 2 months or less frequently, more preferably once every 3 months or less frequently, even more preferably once every 4 months or less frequently, even more preferably once every 5 months or less frequently, or even more preferably once every 6 months or less frequently. Most preferred is once every month.

Preferably, the IL-1β compounds of the invention are administered parenterally, intravenously, e.g. into the antecubital or other peripheral vein, intramuscularly, or subcutaneously. In addition, antibody of the invention may be administered locally, e.g. direct intravitreal injection, subconjunctival injection, subtenon injection, peribulbar injection, intra-ocular implantable device and topical (ointment or eye drop) application.

Pharmaceutical compositions of the invention may be manufactured in conventional manner. A composition according to the invention is preferably provided in lyophilized form. For immediate administration it is dissolved in a suitable aqueous carrier, for example sterile water for injection or sterile buffered physiological saline. If it is considered desirable to make up a solution of larger volume for administration by infusion rather as a bolus injection, it is advantageous to incorporate human serum albumin or the patient's own heparinised blood into the saline at the time of formulation. The presence of an excess of such physiologically inert protein prevents loss of antibody by adsorption onto the walls of the container and tubing used with the infusion solution. If albumin is used, a suitable concentration is from 0.5 to 4.5% by weight of the saline solution.

The invention is further described by way of illustration in the following Examples.

### EXAMPLES

### Example 1: ACZ885

Structure and making of ACZ885 are e.g. described in WO 02/16436. In short, the amino-terminal sequences of heavy and light chain variable domains and the corresponding DNA sequences are given in SEQ ID NO:1 and SEQ ID NO:2 below, in which the CDRs are shown in *Italic* and underlined type.

### ACZ885 Heavy chain variable region SEQ ID NO: 1

TCAG
Q - 1

GTGCAGCTGGTGGAGTCTGGGGGAGGCGTGGTCCAGCCTGGGAGGTCCCTGAGACTCTCC
V Q L V E S G G G V V Q P G R S L R L S - 21

TGTGCAGCGTCTGGATTCACCTTCAGTGTTTATGGCATGAACTGGGTCCGCCAGGCTCCA
C A A S G F T F S V Y G M N W V R Q A P - 41

GGCAAGGGGCTGGAGTGGGTGGCAATTATTTGGTATGATGGAGATAATCAATACTATGCA
G K G L E W V A I I W Y D G D N Q Y Y A - 61

GACTCCGTGAAGGGCCGATTCACCATCTCCAGAGACAATTCCAAGAACACGCTGTATCTG
D S V K G R F T I S R D N S K N T L Y L - 81

CAAATGAACGGCCTGAGAGCCGAGGACACGGCTGTGTATTATTGTGCGAGAGATCTTAGG
Q M N G L R A E D T A V Y Y C A R D L R - 101

ACTGGGCCTTTTGACTACTGGGGCCAGGGAACCCTGGTCACCGTCTCCTC
T G P F D Y W G Q G T L V T V S S 118

### ACZ885 Light chain variable region SEQ ID NO:2

TGAA
E - 1

ATTGTGCTGACTCAGTCTCCAGACTTTCAGTCTGTGACTCCAAAGGAGAAAGTCACCATC
I V L T Q S P D F Q S V T P K E K V T I - 21

ACCTGCCGGGCCAGTCAGAGCATTGGTAGTAGCTTACACTGGTACCAGCAGAAACCAGAT
T C R A S Q S I G S S L H W Y Q Q K P D - 41

CAGTCTCCAAAGCTCCTCATCAAGTATGCTTCCCAGTCCTTCTCAGGGGTCCCCTCGAGG
Q S P K L L I K Y A S Q S F S G V P S R - 61

TTCAGTGGCAGTGGATCTGGGACAGATTTCACCCTCACCATCAATAGCCTGGAAGCTGAA
F S G S G S G T D F T L T I N S L E A E - 81

GATGCTGCAGCGTATTACTGTCATCAGAGTAGTAGTTTACCATTCACTTTCGGCCCTGGG
D A A A Y Y C H Q S S S L P F T F G P G - 101

ACCAAAGTGGATATCAAA - 107
T K V D I K

### Example 2: Biochemical and Biological Data of ACZ885

The monoclonal antibody ACZ 885 is found to neutralize the activity of interleukin-1β in vitro. The monoclonal antibody is further characterized for its binding to recombinant human IL-1β by surface plasmon resonance analysis. The mode of neutralization is assessed by competitive binding studies with soluble IL-1 receptors. The biological activity of the antibody ACZ 885 towards recombinant and naturally produced IL-1β is determined in primary human cell, responsive to stimulation by IL-1β.

### Determination of dissociation equilibrium constant

The association and dissociation rate constants for the binding of recombinant human IL-1β to ACZ885 are determined by surface plasmon resonance analysis. ACZ885 is immobilized, and binding of recombinant IL-1β in a concentration range from 1 to 4 nM is measured by surface plasmon resonance. The chosen format represents a monovalent interaction and thus permits treating the binding event of IL-1β to ACZ885 according to a 1:1 stoichiometry. Data analysis is performed using the BIAevaluation software.

| | **kₒₙ** **[10⁵/Ms]** | **k_{off}** **[10⁻⁵/s]** | **K_{D}** **[pM]** | |
|---|---|---|---|---|
| ACZ885 | 11.0 +/- 0.23 | 3.3+/-0.27 | **30.5+/-2.6** | n=22 |

### Conclusion: ACZ885 binds to recombinant human IL-1β with very high affinity.

### Example 3: Clinical trial with ACZ885 (general aspects)

In order to asses the suitability of an IL-1β compound, e.g. ACZ885, an open-label, single center dose titration study of ACZ885 (human anti-IL-1β monoclonal antibody) to assess the clinical efficacy, safety, pharmacokinetics and pharmacodynamics in patients with an ophthalmic disease or disorder contemplated above is conducted.
Patients are treated by a single dose infusion of ACZ885 (10 mg/kg i.v.). Clinical response is measured by improvement of symptoms (e.g., visual acuity, ocular irritation and inflammation, ocular pain) and improvement of ocular signs (e.g., the intensity of eyelid redness and sweeling (the signs of blepharitis); the number of inflammatory cells and amount of exudate in the anterior chamber or the vitreous; the number, size, and intensity of inflammatory lesions in the conjunctiva, iris, retina, choroid, or sclera; swelling of the retina and specifically the macula, also called macular edema, as measured with ocular coherence tomography; and the quantity of new blood vessels growing in the cornea, the iris, or from the retina or optic nerve) and by lowering of acute phase proteins serum amyloid protein (SAA) and c-reactive protein (CRP) in the blood. In addition, response to treatment is assessed by the analysis of mRNA obtained from peripheral blood cells. A second treatment (1mg/kg i.v.) is given after re-appearance of clinical symptoms.

Results: Clinical remission of symptoms (fever, rash, conjunctivitis) within 3 days, and decrease of CRP and SAA to normal range (< 10 mg/L) in patients. Clinical remission of symptoms with first infusion lasts for at least 134 days, typically between 160 and 200 days. Upon second treatment with lower dose, patients respond with improvement of symptoms and normalization of acute phase proteins.
Analysis of mRNA obtained from peripheral blood cells demonstrates downregulation of the transcription of IL-1b and IL-1b-induced genes within 24h upon treatment with ACZ885. This suggests that ACZ885 is capable to interrupt a positive feedback loop in vivo which leads to self-sustained overproduction of IL-1b in these patients. This contention is also supported by initial characterization of PK/PD effects of ACZ885 which demonstrates the blockade of production of IL-1b upon treatment with ACZ885 in these patients. This particular ability of ACZ885 may contribute (be causal) for its long-lasting clinical effect.

### Example 4: Clinical trial with ACZ885 in ophthalmic diseases

In order to asses the suitability of an IL-1β compound, e.g. ACZ885, a double-masked, multi-center study of ACZ885 (human anti-IL-1β monoclonal antibody) to assess the clinical efficacy and safety in patients with wet age-related macular degeneration (wet AMD) compared to ranibizumab (human VEGF monocolonal fragment antibody) is conducted.
Wet age-related macular degeneration patients are treated with a single dose of ACZ885 (10 mg/kg i.v. infusion) or ranibizumab (300mg intravitreal injection). Clinical response is measured at Month 1 by improvement of symptoms (e.g. best corrected visual acuity) and clinical changes in retinal thickness, choroidal neovascular lesion size and vascular leakage after ACZ885 administration. In addition, systemic markers (e.g. Interleukin-6, c-reactive protein, and tumor necrosis factor- alpha) are measured as exploratory endpoints to assess association with disease progression. After Month 1, the efficacy study is terminated and patients who fulfill the following criteria are treated with ranibizumab as needed (determined by the investigator): Loss of more than 5 letters of best corrected visual acuity from baseline or a reduction of retinal thickness as measured by optical coherence tomography of less than 50 microns. All patients will be followed up to Month 6 to evaluate safety of ACZ885.

Results: It is observed that i.v. infusion of ACZ885 is safe and well tolerated. No ocular adverse effects are observed. Furthermore, retinal thickness as measured by optical coherence tomography is also reduced to clinically significant levels after Month 1.

### Example 5: Animal models to test efficacy of IL-1β antibody:

Due to species specificity of ACZ885, a surrogate murine IL-1β antibody is used to evaluate efficacy of IL-1β compounds in different animal models of disease.
I. Laser-induced Choroidal neovascularization in mice: Mice do not develop age-related macular degeneration, however, choroidal neovascularization resembling human AMD can be induced by using a laser to burn focal disruptions in Bruch's membrane. This injury stimulates the abnormal growth of underlying choroidal capillaries into the RPE layer and subretinal space leading to choroidal neovascularization. Disruption of Bruch's membrane is common to all forms of choroidal neovascularization (CNV), including those associated with the wet form of AMD. Surrogate IL-1β antibodies are administered either intravenously, subcutaneously or intravitreally to prevent or treat the choroidal neovascularization produced by the laser. Choroidal neovascularization is then assessed by preparing retinal flat mounts to visualize the size of choroidal neovascularization as well as vascular leakage. It is observed that all types of administration of IL-1 beta antibody results in a significant decrease in the size of choroidal neovascularization as well as area of vascular leakage.
II. Streptazotocin induced diabetic retinopathy in mice: diabetic retinopathy is induced in mice by administering streptazotocin. Streptazotocin destroys β cells of the pancreas, inducing a diabetic state. With time, mice develop diabetic retinopathy. Surrogate IL-1β antibodies are administered either intravenously, subcutaneously or intravitreally to prevent or treat the developed diabetic retinopathy. Early diabetic retinopathy is assessed by measuring retinal vessel reactivity to increased oxygen as measured by functional magnetic resonance imaging. It is observed that all types of administration of IL-1beta antibody results in increased vessel reactivity as compared to no treatment.
III. Laser induced choroidal neovascularization in IL-1β knock out mice: IL-1β knock out mice are used to validate the role of IL-1β in the wet AMD model, laser-induced CNV in mice. Inhibition of choroidal neovascularization due to the lack of IL-1β is assessed by preparing retinal flat mounts to visualize the size of choroidal neovascularization as well as vascular leakage. It is observed that in IL-beta knock out mice, laser induced CNV is significantly reduced as compared to WT mice. As a result, vascular leakage is also significantly reduced in the IL-1 beta KO mice.
IV. Oxygen induced retinopathy (OIR): OIR is produced by transiently exposing mouse pups to increased ambient oxygen (hyperoxia), resulting in obliteration of the developing microvasculature within the central retina. Subsequent return of the animals to room air results in relatively hypoxic conditions in the retina, which in turn stimulates retinal neovascularization that shares features with diabetic retinopathy, retinopathy of prematurity and other ischemic retinopathies. Surrogate IL-1β antibodies are administered either intravenously, subcutaneously or intravitreally to prevent or treat retinal neovascularization. Retinal neovascularization is assessed by preparing retinal flat mounts to visualize the size of retinal neovascularization. It is observed that all types of administration of IL-1beta antibody results in a significant decrease in the size of retinal neovascularization as compared to untreated mice.
   IL-1β induced retinal inflammation: Direct intravitreal injection of human IL-1β induces ocular neovascularization, retinal inflammation and blood retinal barrier breakdown. Intravenous or subcutaneous administration of ACZ885 inhibits and regresses ocular neovascularization, retinal inflammation and blood retinal barrier breakdown as assessed by immunohistochemical analysis.

## Claims

1. Use of a IL-1 compound in the manufacture of a medicament for the treatment of ophthalmic diseases or disorders.

2. A method for treatment of ophthalmic diseases or disorders in a patient in need thereof which comprises administering to the patient an effective amount of an IL-1 compound.

3. A pharmaceutical composition for use in the treatment of ophthalmic diseases or disorders comprising an IL-1 compound, in combination with a pharmaceutically acceptable excipient, diluent or carrier.

4. The use, method for treatment or pharmaceutical composition according to any of the preceding claims 1, 2 or 3, wherein said IL-β compound is an IL-1, IL-1α or IL-1β compound, preferably an IL-1α or IL-1β compound, and more preferably an IL-1β compound.

5. The use, method for treatment or pharmaceutical composition according to any of the preceding claims 1, 2, 3, or 4, wherein said IL-1 compound is in particular a IL-1β binding molecule which comprises an antigen binding site comprising at least one immunoglobulin heavy chain variable domain (V_{H}) which comprises in sequence hypervariable regions CDR1, CDR2 and CDR3, said CDR1 having the amino acid sequence Val-Tyr-Gly-Met-Asn, said CDR2 having the amino acid sequence Ile-Ile-Trp-Tyr-Asp-Gly-Asp-Asn-Gln-Tyr-Tyr-Ala-Asp-Ser-Val-Lys-Gly, and said CDR3 having the amino acid sequence Asp-Leu-Arg-Thr-Gly-Pro; and direct equivalents thereof.

6. The use, method for treatment or pharmaceutical composition according to any of the preceding claims 1 to 5, wherein said IL-1 compound is in particular an IL-1β binding molecule which comprises both heavy (V_{H}) and light chain (V_{L}) variable domains in which said IL-1β binding molecule comprises at least one antigen binding site comprising:
a) an immunoglobulin heavy chain variable domain (V_{H}) which comprises in sequence hypervariable regions CDR1, CDR2 and CDR3, said CDR1 having the amino acid sequence Val-Tyr-Gly-Met-Asn, said CDR2 having the amino acid sequence Ile-Ile-Trp-Tyr-Asp-Gly-Asp-Asn-Gln-Tyr-Tyr-Ala-Asp-Ser-Val-Lys-Gly, and said CDR3 having the amino acid sequence Asp-Leu-Arg-Thr-Gly-Pro, and
b) an immunoglobulin light chain variable domain (V_{L}) which comprises in sequence hypervariable regions CDR1', CDR2' and CDR3', said CDR1' having the amino acid sequence Arg-Ala-Ser-Gln-Ser-Ile-Gly-Ser-Ser-Leu-His, said CDR2' having the amino acid sequence Ala-Ser-Gln-Ser-Phe-Ser, and said CDR3' having the amino acid sequence Gln-Gln-Arg-Ser-Asn-Trp-Met-Phe-Pro;
and direct equivalents thereof.

7. The use, method for treatment or pharmaceutical composition according to any of the preceding claims 1 to 6, wherein said IL-1 compound is a IL-1β binding molecule which comprises at least one antigen binding site comprising either a first domain having an amino acid sequence substantially identical to that shown in SEQ ID NO: 1 and a second domain having an amino acid sequence substantially identical to that shown in SEQ ID NO:2.

8. The use, method for treatment or pharmaceutical composition according to any of the preceding claims 1 to 7, wherein said ophthalmic disease or disorder is selected from: Wet age-related macular degeneration (wet AMD), dry age-related macular degeneration (dry AMD), diabetic macular edema (DME), cystoid macular edema (CME), non-proliferative diabetic retinopathy (NPDR), proliferative diabetic retinopathy (PDR), cystoid macular edema, vasculitis (e.g. central retinal vein occlusion), papilloedema, retinitis, conjunctivitis, uveitis, choroiditis, multifocal choroiditis, ocular histoplasmosis, blepharitis, dry eye (Sjögren's disease) and other ophthalmic diseases and disorders involving inflammation wherein the eye disease or disorder is associated with ocular neovascularization, vascular leak, and/or retinal edema; preferably from wet AMD, dry AMD, CME, DME, NPDR, PDR, blepharitis, dry eye and uveitis; more preferably from wet AMD, dry AMD, blepharitis, and dry eye, more preferably from CME, DME, NPDR and PDR; more preferably from blepharitis, and dry eye; in particular from wet AMD and dry AMD; and especially wet AMD.

9. The use, method for treatment or pharmaceutical composition according to any of the preceding claims 1 to 8 wherein said IL-1 compound is applied once every 1 week or less frequently.

10. The use, method for treatment or pharmaceutical composition according to any of the preceding claims 1 to 8 wherein the application of said IL-compound is subcutaneous injection, intravenous injection/infusion, intramuscular injection, intravitreal injection, intra-ocular implant for extended release, subtenon injection or implant, subconjunctival injection, juxtascleral injection or implant, peribulbar injection, and topical (ointment or eye drop).

11. The use, method for treatment or pharmaceutical composition according to any of the preceding claims wherein said IL-1 compound is an IL-1β binding compound, or an IL-1β receptor binding compound, or a compound decreasing protein levels of either IL-1β or IL-1β preceptor.

12. The use, method for treatment or pharmaceutical composition according to any of the preceding claims wherein said IL-1 compound is an IL-1β antibody.
